# EUROPEAN PATENT SPECIFICATION

(11) **EP 2 451 449 B1**
(45) Date of publication and mention of the grant of the patent: **07.05.2014**
(21) Application number: 10731897.4
(22) Date of filing: 08.07.2010
(51) Int. Cl.: A61K 31/165, A61K 38/21, A61P 25/00

(54) **USE OF THE COMBINATION OF TERIFLUNOMIDE AND INTERFERON BETA FOR TREATING MULTIPLE SCLEROSIS**
VERWENDUNG DER KOMBINATION AUS TERIFLUNOMID UND INTERFERON BETA ZUR BEHANDLUNG DER MULTIPLEN SKLEROSE
UTILISATION D'UNE COMBINAISON DE TERIFLUNOMIDE ET D'INTERFÉRON BÊTA POUR LE TRAITEMENT DE LA SCLÉROSE EN PLAQUES

(30) Priority: 10.07.2009 EP 09305669; 14.07.2009 US 225342 P; 14.12.2009 US 286148 P; 03.06.2010 US 350957 P
(43) Date of publication of application: 16.05.2012
(73) Proprietor: Sanofi-Aventis U.S. LLC, Bridgewater, NJ 08807 (US)
(72) Inventor: BYRNES, William, Bridgewater, New Jersey 08807 (US); DOUILLET, Patrice, F-75014 Paris (FR); FRANGIN, Gerald, F-75014 Paris (FR)
(74) Representative: Weiss, Wolfgang
(86) International application number: PCT/US2010/041274
(87) International publication number: WO 2011/005907

(56) References cited:
- EP-A- 1 935 416
- O'CONNOR P W ET AL: "A Phase II study of the safety and efficacy of teriflunomide in multiple sclerosis with relapses." NEUROLOGY 28 MAR 2006, vol. 66, no. 6, 28 March 2006 (2006-03-28) , pages 894-900, XP002556504 ISSN: 1526-632X
- PILZ G ET AL: "Modern multiple sclerosis treatment - what is approved, what is on the horizon" DRUG DISCOVERY TODAY, ELSEVIER, RAHWAY, NJ, US, vol. 13, no. 23-24, 1 December 2008 (2008-12-01), pages 1013-1025, XP025658656 ISSN: 1359-6446 [retrieved on 2008-09-25]
- TALLANTYRE E ET AL: "Spotlight on teriflunomide." INTERNATIONAL MS JOURNAL / MS FORUM JUN 2008, vol. 15, no. 2, June 2008 (2008-06), pages 62-68, XP002556505 ISSN: 1352-8963
- WARNKE CLEMENS ET AL: "Review of teriflunomide and its potential in the treatment of multiple sclerosis." NEUROPSYCHIATRIC DISEASE AND TREATMENT 2009, vol. 5, 2009, pages 333-340, XP002556506 ISSN: 1176-6328

## Description

### Field of the Invention

This invention is related to the use of the combination of teriflunomide and interferon beta thereof, for the preparation of a medicament for use in treating multiple sclerosis.

### Background of the Invention

Multiple sclerosis (MS) is a debilitating, inflammatory, neurological illness characterized by demyelination of the central nervous system. The disease primarily affects young adults with a higher incidence in females. Symptoms of the disease include fatigue, numbness, tremor, tingling, dysesthesias, visual disturbances, dizziness, cognitive impairment, urologic dysfunction, decreased mobility, and depression. Four types classify the clinical patterns of the disease: relapsing-remitting, secondary progressive, primary-progressive and progressive-relapsing (S.L. Hauser and D.E. Goodkin, Multiple Sclerosis and Other Demyelinating Diseases in Harrison's Principles of Internal Medicine, 14th Edition, vol. 2, Mc Graw-Hill, 1998, pp. 2409-2419).

The exact etiology of MS is unknown; however, it is strongly suspected that the demyelination characteristic of the disease is the result of an autoimmune response perhaps triggered by an environmental insult, e.g. a viral infection. Specifically, it is hypothesized that MS is caused by a T-cell-mediated, autoimmune inflammatory reaction. The autoimmune basis is strongly supported by the fact that antibodies specific to myelin basic protein (MBP) have been found in the serum and cerebrospinal fluid of MS patients and these antibodies along with T-cells that are reactive to MBP and other myelin proteolipids increase with disease activity. Furthermore, at the cellular level it is speculated that T-cell proliferation and other cellular events, such as activation of B cells and macrophages and secretion of cytokines accompanied by a breakdown of the blood-brain barrier can cause destruction of myelin and oligodendrocytes. (R.A. Adams, M.V. Victor and A.H. Ropper eds, Principles of Neurology, Mc Graw-Hill, New York, 1997, pp.903-921). Progressive MS (primary and secondary) may be based on a neurodegenerative process occurring with demyelination.

The use of (Z)-2-cyano-3-hydroxy-but-2-enoic acid-(4'-trifluoromethylphenyl)-amide (also known as teriflunomide, Formula I) for treating multiple sclerosis has been disclosed in U.S. Patent No. 6,794,410. Although aforesaid patent discloses that teriflunomide may possibly be combined with another compound known to be effective for treating multiple sclerosis to treat the disease, no specific combination is disclosed to show such efficacy and safety in treating multiple sclerosis.

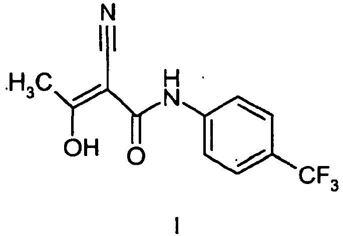

### Summary of the Invention

This invention is related to teriflunomide for use in treating replapsing-remitting form of multiple sclerosis, in a.patient in need thereof, said use comprising administering to the patient about 7 mg or about 14 mg teriflunomide, and a pharmaceutically effective amount of interferon beta-1a or 1b.

### Detailed Description of the Invention

As used above, and throughout the description of the invention, the following terms, unless otherwise indicated, shall be understood to have the following meanings:
"Clinically proven effective" mean that the results of clinical trial are statistically significant, i.e., the results of the clinical trial are not likely to be due to chance with an alpha level less than 0.05.
"Patient" means mammals, particularly humans.
"Pharmaceutically effective amount" means an amount of a compound/composition according to the present invention effective in producing the desired therapeutic effect.
"Stable dose of interferon beta-1a or 1b" means administering, for example, about 30 mcg beta-1a once a week, particularly intramuscularly, or about 22 mcg beta-1a three times a week, particularly subcutaneously, or about 44 mcg beta-1a three times a week, particularly subcutaneously, or 0.25 mg beta-1b every other day, particularly subcutaneously.
"Treat" or "treating" means to alleviate symptoms, eliminate the causation of the symptoms either on a temporary or permanent basis, or to prevent or slow the appearance of symptoms of the named disorder or condition.

One particular embodiment of the invention is teriflunomide for use in treating relapsingremitting form of multiple sclerosis, in a patient in need thereof, said use comprising administering to the patient about 7 mg or about 14 mg teriflunomide once a day, and a stable dose of interferon beta-1a or 1b.

Another particular embodiment of the invention is teriflunomide for use in treating relapsingremitting form of multiple sclerosis, in a patient in need thereof, said use comprising concurrently administering to the patient about 7 mg or about 14 mg teriflunomide once a day, and a stable dose of interferon beta-1 a or 1b.

Another particular embodiment of the invention is related to teriflunomide for use in treating relapsing-remitting form of multiple sclerosis, in a patient in need thereof, said use comprising administering to the patient about 7 mg or about 14 mg teriflunomide once a day, and a stable dose of interferon beta-1 a or 1b in a clinically proven effective method.

Another particular embodiment of the invention is related to teriflunomide for use in treating relapsing-remitting form of multiple sclerosis, in a patient in need thereof, said use comprising administering to the patient about 7 mg teriflunomide once a day and about 30 mcg beta-1a once a week.

Another particular embodiment of the invention is related to teriflunomide for use in treating relapsing-remitting form of multiple sclerosis, in a patient in need thereof, said use comprising administering to the patient about 7 mg teriflunomide once a day and about 22 mcg beta-1a three times a week.

Another particular embodiment of the invention is related to teriflunomide for use in treating relapsing-remitting form of multiple sclerosis, in a patient in need thereof, said use comprising administering to the patient about 7 mg teriflunomide once a day and about 44 mcg beta-1 a three times a week.

Another particular embodiment of the invention is related to teriflunomide for use in treating relapsing-remitting form of multiple sclerosis, in a patient in need thereof, said use comprising administering to the patient about 7 mg teriflunomide once a day and about 0.25 mg beta-1 b every other day.

Another particular embodiment of the invention is related to teriflunomide for use in treating relapsing-remitting form of multiple sclerosis, in a patient in need thereof, said use comprising administering to the patient about 14 mg teriflunomide once a day and about 30 mcg beta-1a once a week.

Another particular embodiment of the invention is related to teriflunomide for use in treating relapsing-remitting form of multiple sclerosis, in a patient in need thereof, said use comprising administering to the patient about 14 mg teriflunomide once a day and about 22 mcg beta-1 a three times a week.

Another particular embodiment of the invention is related to teriflunomide for use in treating multiple sclerosis, in a patient in need thereof, said use comprising administering to the patient about 14 mg teriflunomide once a day and about 44 mcg beta-1a three times a week.

Another particular embodiment of the invention is related to teriflunomide for use in treating relapsing-remitting form of multiple sclerosis, in a patient in need thereof, said use comprising administering to the patient about 14 mg teriflunomide once a day and about 0.25 mg beta-1 b every other day.

Another particular embodiment of the invention is related to teriflunomide for use in treating relapsing-remitting form of multiple sclerosis, said use comprising administering teriflunomide orally.

Another particular embodiment of the invention is related to teriflunomide for use in treating relapsing-remitting form of multiple sclerosis, said use comprising administering the interferon beta-1a intramuscularly or subcutaneously.

Another particular embodiment of the invention is related teriflunomide for use in treating relapsing-remitting form of multiple scierosis, said use comprising administering the interferon beta-1b subcutaneously.

Another particular embodiment of the invention is related to a teriflunomide for use in reducing the number of T1-Gd lesions in a patient afflicted with relapsing-remitting form of multiple sclerosis, said use comprising administering to the patient about 7 mg or about 14 mg teriflunomide, and a pharmaceutically effective amount of interferon beta-1a or 1 b.

Another particular embodiment of the invention is related to teriflunomide for use in reducing the number of T1-Gd lesions in a patient afflicted with relapsing-remitting form of multiple sclerosis, said use comprising administering to the patient about 7 mg or about 14 mg teriflunomide once a day, and a stable dose of interferon beta-1a or 1 b.

Another particular embodiment of the invention is related to teriflunomide for use in reducing the number of T1-Gd lesions in patients afflicted with relapsing-remitting form of multiple sclerosis, said use comprising administering to the patient about 7 mg or about 14 mg teriflunomide once a day, and a stable dose of interferon beta-1a or 1b in a clinically proven effective method.

Another particular embodiment of the invention is related to teriflunomide for use in reducing the number of T1-Gd lesions in patients afflicted with relapsing-remitting form of multiple sclerosis, said use comprising concurrently administering to the patient about 7 mg or about 14 mg teriflunomide once a day, and a stable dose of interferon beta-1a or 1b in a clinically proven effective method.

Another particular embodiment of the invention is related to teriflunomide for use in reducing the number of T1-Gd lesions in a patient afflicted with relapsing-remitting form of multiple sclerosis, said use comprising administering to the patient about 7 mg or about 14 mg teriflunomide once a day, and a stable dose of interferon beta-1a or 1 b, wherein more number of T1-Gd lesions is reduced in the patient than in a patient treated by a stable dose of interferon beta-1a or 1 b alone.

Another particular embodiment of the invention is related to teriflunomide for use in reducing numbers of T1-Gd lesions in patients afflicted with relapsing-remitting form of multiple sclerosis, said use comprising administering to the patients about 7 mg or about 14 mg teriflunomide once a day, and a stable dose of interferon beta-1a or 1b in a clinically proven effective method wherein more numbers of T1-Gd lesions are reduced in the patients treated by the method than in patients treated by a stable dose of interferon beta-1a or 1 b alone

Another particular embodiment of the invention is related to teriflunomide for use in reducing the number of T1-Gd lesions in patients afflicted with relapsing-remitting form of multiple sclerosis, said use comprising administering to the patient about 7 mg teriflunomide once a day, and a stable dose of interferon beta-1a or 1b, wherein the number of T1-Gd lesions in the patients is reduced about 82.6% to about 84.6% comparing to the number of lesions in patients treated by a stable dose of interferon beta-1a or 1 b alone.

Another particular embodiment of the invention is related to teriflunomide for use in reducing the number of T1-Gd lesions in patients afflicted with relapsing-remitting form of multiple sclerosis, said use comprising administering to the patient about 14 mg teriflunomide once a day, and a stable dose of interferon beta-1 a or 1 b, wherein the number of T1-Gd lesions in the patients is reduced about 82.8% to about 84.4% comparing to the number of lesions in patients treated by a stable dose of interferon beta-1a or 1 b alone.

Another particular embodiment of the invention is related to teriflunomide for use in reducing the volume of T1-Gd lesions in a patient afflicted with relapsing-remitting form of multiple sderosis, said use comprising administering to the patient about 7 mg or about 14 mg teriflunomide, and a pharmaceutically effective amount of interferon beta-1a or 1 b.

Another particular embodiment of the invention is related to teriflunomide for use in reducing the volume of T1-Gd lesions in a patient afflicted with relapsing-remitting form of multiple sclerosis, said use comprising administering to the patient about 7 mg or about 14 mg teriflunomide once a day, and a stable dose of interferon beta-1a or 1 b.

Another particular embodiment of the invention is related to teriflunomide for use in reducing the volume of T1-Gd lesions in patients afflicted with relapsing-remitting form of multiple sclerosis, said use comprising administering to the patients about 7 mg or about 14 mg teriflunomide once a day, and a stable dose of interferon beta-1a or 1b in a clinically proven effective method.

Another particular embodiment of the invention is related to teriflunomide for use in reducing the volume of T1-Gd lesions in patients afflicted with relapsing-remitting form of multiple sclerosis, said use comprising concurrently administering to the patients about 7 mg or about 14 mg teriflunomide once a day, and a stable dose of interferon beta-1a or 1b in a clinically proven effective method.

Another particular embodiment of the invention is related to teriflunomide for use in reducing the volume of T1-Gd lesions in a patient afflicted with relapsing-remitting form of multiple sclerosis, said use comprising administering to the patient about 7 mg or about 14 mg teriflunomide once a day, and a stable dose of interferon beta-1 a or 1 b, wherein more volume of T1-Gd lesions is reduced in the patient treated by the method than in a patient treated by a stable dose of interferon beta-1 a or 1 b alone.

Another particular embodiment of the invention is related to teriflunomide for use in reducing the volume of T1-Gd lesions in patients afflicted with relapsing-remitting form of multiple sclerosis, said use comprising administering to the patients about 14 mg teriflunomide once a day, and a stable dose of interferon beta-1a or 1b, wherein more volumes of T1-Gd lesions are reduced in the patients than in patients treated by a stable dose of interferon beta-1a or 1b alone in a clinically proven effective method.

Another particular embodiment of the invention is related to teriflunomide for use in reducing the volume of T1-Gd lesions in patients afflicted with relapsing-remitting form of multiple sclerosis, said use comprising administering to the patient about 14 mg teriflunomide once a day, and a stable dose of interferon beta-1a or 1b, wherein the volume of T1-Gd lesions in the patients treated by the method is reduced about 64.7% to about 70.6% comparing to the volume of lesions in patients treated by a stable dose of interferon beta-1a or 1b alone.

Another particular embodiment of the invention is related to teriflunomide for use in treating relapsing-remitting form of multiple sclerosis in patients in need thereof said use comprising administering to the patients 7 mg teriflunomide once a day and a stable dose of interferon-beta 1a or 1b, wherein about 69.4% of the patients are free of T1-Gd lesions after about 24-week treatment.

Another particular embodiment of the invention is related to teriflunomide for use in treating relapsing-remitting form of multiple sclerosis in patients in need thereof said use comprising administering to the patients 7 mg teriflunomide once a day and a stable dose of interferon-beta 1aor 1b, wherein about 69.4% of the patients are free of T1-Gd lesions after about 48-week treatment.

Another particular embodiment of the invention is related to teriflunomide for use in treating relapsing-remitting form of multiple sclerosis in patients in need thereof said use comprising administering to the patients 14 mg teriflunomide once a day and a stable dose of interferon-beta 1 aor 1 b, wherein about 81.6% of the patients are free of T1-Gd lesions after about 24-week treatment.

Another particular embodiment of the invention is related to teriflunomide for use in treating relapsing-remitting form of multiple sclerosis in patients in need thereof said use comprising administering to the patients 14 mg teriflunomide once a day and a stable dose of interferon-beta 1 aor 1 b, wherein about 76.3% of the patients are free of T1-Gd lesions after about 48-week treatment.

Another particular embodiment of the invention is related to the use of about 7 mg or about 14 mg teriflunomide for the preparation of a medicament for treating relapsing-remitting form of multiple sclerosis, wherein said medicament is administered to a patient in combination of a pharmaceutically effective amount of interferon beta 1 a or 1 b.

Another particular embodiment of the invention is related to the use of about 7 mg or about 14 mg teriflunomide for the preparation of a medicament for treating relapsing-remitting form of multiple sclerosis, wherein said medicament is administered once a day to a patient in combination of a stable dose of interferon beta 1 a or 1 b.

Another particular embodiment of the invention is related to the use of about 7 mg or about 14 mg teriflunomide for the preparation of a clinically proven effective medicament for treating relapsing-remitting form of multiple sclerosis, wherein said medicament is administered once a day in combination of a stable dose of interferon beta 1a or 1b.

Another particular embodiment of the invention is related to the use of about 7 mg or about 14 mg teriflunomide for the preparation of a clinically proven effective medicament for treating relapsing-remitting form of multiple sclerosis, wherein said medicament is administered once a day to a patient who is concurrently on a stable dose of interferon beta 1 a or 1 b.

Another particular embodiment of the invention is related to the use of about 7 mg or about 14 mg teriflunomide for the preparation of a medicament for reducing the number of T1-Gd lesions in a patient afflicted with relapsing-remitting form of multiple sclerosis, wherein said medicament is administered once a day to the patient in combination of a pharmaceutically effective amount of interferon beta 1 a or 1 b.

Another particular embodiment of the invention is related to the use of about 7 mg or about 14 mg teriflunomide for the preparation of a medicament for reducing the number of T1-Gd lesions in a patient afflicted with relapsing-remitting form of multiple sclerosis, wherein said medicament is administered once a day to the patient in combination of a stable dose of interferon beta 1 a or 1 b.

Another particular embodiment of the invention is related to the use of about 7 mg or about 14 mg teriflunomide for the preparation of a clinically proven effective medicament for reducing the number of T1-Gd lesions in a patient afflicted with relapsing-remitting form of multiple sclerosis, wherein said medicament is administered once a day to the patient in combination of a stable dose of interferon beta 1a or 1b.

Another particular embodiment of the invention is related to the use of about 7 mg or about 14 mg teriflunomide for the preparation of a medicament for reducing the number of T1-Gd lesions in a patient afflicted with relapsing-remitting form of multiple sclerosis, wherein said medicament is administered once a day to the patient who is concurrently on a stable dose of interferon beta 1 a or 1 b.

Another particular embodiment of the invention is related to the use of about 7 mg or about 14 mg teriflunomide for the preparation of a medicament for reducing the number of T1-Gd lesions in a patient afflicted with relapsing-remitting form of multiple sclerosis, wherein said medicament is administered once a day to the patient in combination of a stable dose of interferon beta 1 a or 1 b such that more number of T1-Gd lesions are reduced in the patient than in a patient treated by a stable dose of interferon beta-1 a or 1 b alone.

Another particular embodiment of the invention is related to the use of about 7 mg or about 14 mg teriflunomide for the preparation of a clinically proven effective medicament for reducing the number of T1-Gd lesions in patients afflicted with relapsing-remitting form of multiple sclerosis, wherein said medicament is administered once a day to the patients in combination of a stable dose of interferon beta 1 a or 1 b such that more number of T1-Gd lesions are reduced in the patients than in patients treated by a stable dose of interferon beta-1a or 1 b alone.

Another particular embodiment of the invention is related to the use of about 7 mg teriflunomide for the preparation of a medicament for reducing the number of T1-Gd lesions in patients afflicted with relapsing-remitting form of multiple sclerosis, wherein said medicament is administered once a day to the patients in combination of a stable dose of interferon beta 1 a or 1 b such that the number of T1-Gd lesions in the patients is reduced about 56% to about 84.6% comparing to the number of lesions in patients treated by a stable dose of interferon beta-1 a or 1 b alone.

Another particular embodiment of the invention is related to the use of about 14 mg teriflunomide for the preparation of a medicament for reducing the number of T1-Gd lesions in patients afflicted with relapsing-remitting form of multiple sclerosis, wherein said medicament is administered once a day to the patients in combination of a stable dose of interferon beta 1 a or 1 b such that the number of T1-Gd lesions in the patients is reduced about 81% to about 82.8% comparing to the number of lesions in patients treated by a stable dose of interferon beta-1a or 1 b alone.

Another particular embodiment of the invention is related to the use of about 7 mg or about 14 mg teriflunomide for the preparation of a medicament for reducing the volume of T1-Gd lesions in a patient afflicted with relapsing-remitting form of multiple sclerosis, wherein said medicament is administered once a day to the patient in combination of a pharmaceutically effective amount of interferon beta 1 a or 1 b.

Another particular embodiment of the invention is related to the use of about 7 mg or about 14 mg teriflunomide for the preparation of a medicament for reducing the volume of T1-Gd lesions in a patient afflicted with relapsing-remitting form of multiple sclerosis, wherein said medicament is administered once a day to the patient in combination of a stable dose of interferon beta 1 a or 1 b.

Another particular embodiment of the invention is related to the use of about 7 mg or about 14 mg teriflunomide for the preparation of a clinically proven effective medicament for reducing the volume of T1-Gd lesions in a patient afflicted with relapsing-remitting form of multiple sclerosis, wherein said medicament is administered once a day to the patient in combination with a stable dose of interferon beta 1 a or 1 b.

Another particular embodiment of the invention is related to the use of about 7 mg or about 14 mg teriflunomide for the preparation of a medicament for reducing the volume of T1-Gd lesions in a patient afflicted with relapsing-remitting form of multiple sclerosis, wherein said medicament is administered once a day to the patient who is concurrently on a stable dose of interferon beta 1 a or 1 b.

Another particular embodiment of the invention is related to the use of about 7 mg or about 14 mg teriflunomide for the preparation of a medicament for reducing the volume of T1-Gd lesions in a patient afflicted with relapsing-remitting form of multiple sclerosis, wherein said medicament is administered once a day to the patient in combination of a stable dose of interferon beta 1 a or 1 b such that more volume of T1-Gd lesions are reduced in the patient than in a patient treated by a stable dose of interferon beta-1 a or 1 b alone.

Another particular embodiment of the invention is related to the use of about 7 mg or about 14 mg teriflunomide for the preparation of a clinically proven effective medicament for reducing the volume of T1-Gd lesions in patients afflicted with relapsing-remitting form of multiple sclerosis, wherein said medicament is administered once a day to the patients in combination of a stable dose of interferon beta 1 a or 1 b such that more volume of T1-Gd lesions are reduced in the patients than in patients treated by a stable dose of interferon beta-1 a or 1 b alone.

Another particular embodiment of the invention is related to the use of about 7 mg teriflunomide for the preparation of a medicament for reducing the volume of T1-Gd lesions in patients afflicted with relapsing-remitting form of multiple sclerosis, wherein said medicament is administered once a day to the patients in combination of a stable dose of interferon beta 1 a or 1 b such that the volume of T1-Gd lesions in the patients is reduced about 46% comparing to the number of lesions in patients treated by a stable dose of interferon beta-1 a or 1 b alone.

Another particular embodiment of the invention is related to the use of about 14 mg teriflunomide for the preparation of a medicament for reducing the number of T1-Gd lesions in patients afflicted with relapsing-remitting form of multiple sclerosis, wherein said medicament is administered once a day to the patients in combination of a stable dose of interferon beta 1 a or 1 b such that the number of T1-Gd lesions in the patients is reduced about 66% comparing to the number of lesions in patients treated by a stable dose of interferon beta-1a or 1 b alone.

Another particular embodiment of the invention is related to the use of about 7 mg teriflunomide for the preparation of a medicament for treating relapsing-remitting form of multiple sclerosis, wherein said medicament is administered once a day to patients in combination of a stable dose of interferon beta 1 a or 1 b such that about 70% of the patients are free of T1-Gd lesions after about 24-week treatment.

Another particular embodiment of the invention is related to the use of about 7 mg teriflunomide for the preparation of a medicament for treating relapsing-remitting form of multiple sclerosis, wherein said medicament is administered once a day to patients in combination of a stable dose of interferon beta 1 a or 1 b such that about 69% of the patients are free of T1-Gd lesions after about 48-week treatment.

Another particular embodiment of the invention is related to the use of about 14 mg teriflunomide for the preparation of a medicament for treating relapsing-remitting form of multiple sclerosis, wherein said medicament is administered once a day to patients in combination of a stable dose of interferon beta 1 a or 1 b such that about 70% of the patients are free of T1-Gd lesions after about 24-week treatment.

Another particular embodiment of the invention is related to the use of about 14 mg teriflunomide for the preparation of a medicament for treating relapsing-remitting form of multiple sclerosis, wherein said medicament is administered once a day to patients in combination of a stable dose of interferon beta 1 a or 1 b such that about 76% of the patients are free of T1-Gd lesions after about 48-week treatment.

It is now clinically proved that the addition of teriflunomide to stable-dosed interferon improves disease control beyond interferon alone, in terms of MRI activity, without added concerns regarding safety and tolerability.

### Examples

The present invention may be better understood by reference to the following nonlimiting Examples, which are exemplary of the invention. They should in no way be construed, however, as limiting the broad scope of the invention.

### Example 1

A placebo-controlled, double-blinded, randomized, 3-parallel group stratified study was conducted in relapsing-remitting multiple sclerosis patient who were concurrently on a stable dose of interferon beta. The dose level of interferon beta was defined as follows:
- Low dose: AVONEX® (interferon beta-1a) 30 mcg once a week intramuscularly, or REBIF® (interferon beta-1a) 22 mcg three times per week subcutaneously.
- High dose: REBIF® 44 mcg three times per week subcutaneously, or BETASERON® (interferon beta-1 b) 0.25 mg every other day subcutaneously.

Around 40 patients were treated in each treatment group (placebo: 41, 7 mg: 37, and 14 mg: 38). A third of patients in each group were treated with low dose interferon beta 1a. The demographic and disease baseline characteristics were generally comparable amount the 3 treatment groups. The mean age of the study population was 40.1 years. The majority of patients were female (69.8%) and had a relapsing-remitting type of MS (87.9%), with a diagnosis of MS since around 8 years ago and around 40% of patients had no relapse in the previous year. The base line Expanded Disability Status Scale (EDSS) score was similar between treatment groups (around 2.5).

### Method:

Safety was evaluated from Treatment Emergent Adverse Event (TEAE), physical examination and laboratory data (e.g. Liver Function Test, every 2-8 weeks), ECG and abdominal ultrasound (24-weeks). Brain MRI (magnetic resonance image) activity, including T1-gadolinium (T1-Gd) with central reading were recorded every 8-week. Relapses were recorded and EDSS was performed at 24-week.

### Results:

Safety: Approximately 90% of patients completed the 24-week treatment period in each group. Treatment was prematurely discontinued for TEAE in one patient in each group. The proportion of patients with TEAE due to increased ALT/SGPT (Alanine Transaminase/Serum Glutamic Pyruvic Transaminase) was higher on 14 mg (28.9%) than on 7 mg (13.5%) or placebo (12.2%). Among them, the proportion of patients with ALT/SGPT greater than 3xULN (three times above the Upper Limit of Normal range for the central laboratory) was low (4.9% in placebo; 0% in 7 mg; 5.3% in 14 mg) with one treatment discontinuation from placebo and one from 14 mg. There was no case of concomitant bilirubin increase. The proportion of patients with TEAE potentially related to immunosuppression (including white blood cell counts, infections and infestations) was slightly higher in the teriflunomide groups (placebo: 32%, 7 mg: 49%, 14 mg: 50%). Among these events upper respiratory tract infections (nasopharyngitis, sinusitis) appeared to be more frequent at 14 mg (23.7%) than placebo (14.6%) and 7 mg (10.8%). None of these events led to treatment discontinuation.

Efficacy: As shown in Tables 1, 2 and 3, the adjusted results of the 24 week study showed that patients taking 7 mg or 14 mg teriflunomide in addition to stable-dose IFN-beta experienced a significant relative risk reduction in the number of gadolinium enhancing T1 (T1-Gd) lesions on brain magnetic resonance imaging, 82.6% (p=0.0009) and 84.4% (p<0.0001), respectively, compared with placebo added to IFN-beta. There was also a trend to a dose-dependent relative reduction of T1-Gd lesion volume in both teriflunomide arms compared to placebo of 67.6% at 7 mg (p=0.1931) and 64.7% at 14 mg (p=0.0072). The proportion of patients free of T1-Gd lesions was higher in both teriflunomide arms (placebo: 57.9%, 7 mg: 69.4%, 14 mg: 81.6%). A few relapses were reported during the 24-week period (5 on placebo, 5 on 7 mg and 2 on 14 mg).

Conclusion: The addition of teriflunomide to stable-dosed interferon beta significantly improved disease control beyond interferon beta alone, as evaluated by T1-gadolinium MRI activity, with acceptable safety and tolerability over 24 weeks of treatment.

**Table 1 - Total number of Gd-enhancing T1-lesions per MRI scan - ITT population**

| | | **teriflunomide** | |
|---|---|---|---|
| | **Placebo+IFN-β (N=41)** | **7 mg teri+IFN-β (N=37)** | **14 mg teri+IFN-β (N=38)** |
| **Baseline** | | | |
| Patients with >=1 Gd-enhancing T1-lesions | | | |
| Yes | 9 (22.5%) | 8 (21.6%) | 8 (21.1%) |
| No | 31 (77.5%) | 29 (78.4%) | 30 (78.9%) |
| | | | |

| Patient Gd-enhancing T1-lesions | | | |
|---|---|---|---|
| N | 40 | 37 | 38 |
| Mean (SD) | 0.725 (1.679) | 0.838 (2.892) | 0.579 (1.734) |
| Median | 0.000 | 0.000 | 0.000 |
| Min : Max | 0.00 : 7.00 | 0.00 : 17.00 | 0.00: 10.00 |
| | | | |

| **Post-baseline** | | | |
|---|---|---|---|
| Patients with >=1 Gd-enhancing T1-lesions | | | |
| Yes | 16 (42.1%) | 11 (30.6%) | 7 (18.4%) |
| No | 22 (57.9%) | 25 (69.4%) | 31 (81.6%) |
| P-value ^{a} | | 0.3416 | 0.0445 |
| | | | |

| Number of Gd-enhancing T1-lesions | | | |
|---|---|---|---|
| 0 | 22 (57.9%) | 25 (69.4%) | 31 (81.6%) |
| 1 | 3 (7.9%) | 8 (22.2%) | 4 (10.5%) |
| 2 | 3 (7.9%) | 0 | 1 (2.6%) |
| 3 | 3 (7.9%) | 1 (2.8%) | 1 (2.6%) |
| >=4 | 7 (18.4%) | 2 (5.6%) | 1 (2.6%) |
| | | | |
| Total number of Gd-enhancing T1-lesions | 84 | 33 | 15 |
| Total number of scans | 113 | 98 | 108 |
| Unadjusted Gd-enhancing T1-lesions per scan ^{b} | 0.743 | 0.337 | 0.139 |
| | | | |

| Adjusted Gd-enhancing T1-lesions per scan ^{c} | | | |
|---|---|---|---|
| Estimate (95% Cl) | 0.570 (0.350, 0.929) | 0.099 (0.041, 0.241) | 0.089 (0.050, 0.159) |
| Relative risk (95% Cl) | | 0.174 (0.062, 0.487) | 0.156 (0.080, 0.304) |
| P-value | | 0.0009 | <.0001 |
| | | | |

| Patient Gd-enhancing T1-lesions per scan ^{d} | | | |
|---|---|---|---|
| N | 38 | 36 | 38 |
| Mean (SD) | 0.776 (1.430) | 0.417 (1.238) | 0.132 (0.376) |
| Median | 0.000 | 0.000 | 0.000 |
| Min : Max | 0.00 : 5.67 | 0.00 : 5.67 | 0.00 : 2.00 |

| | | | |
|---|---|---|---|
| ^{a} From Fisher's exact test. ^{b} The total number of Gd-enhancing T1-lesion that occurred during the study divided by the total number of scans during the study. ^{c} Poisson model with the total number of Gd-enhancing T1-lesions as the response variable, baseline number of Gd-enhancing T1-lesions, treatment, IFN-β dose strata and region as covariates, and log-transformed number of scans as an offset variable. ^{d} The number of Gd-enhancing T1-lesions for each patient divided by the number of scans for that patient. | | | |

**Table 2 - Total volume (ml) of Gd-enhancing T1-lesions per MRI scan - ITT population**

| | | **teriflunomide** | |
|---|---|---|---|
| | **Placebo+IFN-β (N=41)** | **7 mg teri+IFN-β (N=37)** | **14 mg teri+IFN-β (N=38)** |
| **Baseline** | | | |
| N | 40 | 37 | 38 |
| Mean (SD) | 0.078 (0.201) | 0.107 (0.344) | 0.047 (0.120) |
| Median | 0.000 | 0.000 | 0.000 |
| Min : Max | 0.00 : 0.94 | 0.00 : 1.67 | 0.00 : 0.54 |
| | | | |

| **Post-baseline** | | | |
|---|---|---|---|
| Gd-enhancing T1-lesions per scan ^{a} | 0.068 | 0.022 | 0.024 |
| | | | |

| Patient Gd-enhancing T1-lesions per scan ^{b} | | | |
|---|---|---|---|
| N | 38 | 36 | 38 |
| Mean (SD) | 0.069 (0.154) | 0.037 (0.149) | 0.022 (0.087) |
| Median | 0.000 | 0.000 | 0.000 |
| Min : Max | 0.00 : 0.67 | 0.00 : 0.87 | 0.00 : 0.50 |
| | | | |
| P-value ^{c} | | 0.1931 | 0.0072 |

| | | | |
|---|---|---|---|
| ^{a} The total volume of Gd-enhancing T1-lesion that occurred during the study divided by the total number of scans during the study. ^{b} The volume of Gd-enhancing T1-lesions for each patient divided by the number of scans for that patient. ^{c} Rank ANCOVA adjusted for IFN-β dose strata, region and ranked baseline volume of Gd-enhancing T1-lesions. | | | |

**Table 3 - Analysis of MS relapse - ITT population**

| | | **teriflunomide** | |
|---|---|---|---|
| | **Placebo+IFN-β (N=41)** | **7 mg teri+IFN-β (N=37)** | **14 mg teri+IFN-β (N=38)** |
| Number of patients with >=1 relapses | | | |
| Yes | 5 (12.2%) | 5 (13.5%) | 2 (5.3%) |
| No | 36 (87.8%) | 32 (86.5%) | 36 (94.7%) |
| | | | |

| Number of relapses | | | |
|---|---|---|---|
| 0 | 36 (87.8%) | 32 (86.5%) | 36 (94.7%) |
| 1 | 5 (12.2%) | 5 (13.5%) | 2 (5.3%) |
| 2 | 0 | 0 | 0 |
| 3 | 0 | 0 | 0 |
| >=4 | 0 | 0 | 0 |
| | | | |
| Total number of relapses | 5 | 5 | 2 |
| Total patient-years followed | 17.9 | 16.4 | 17.3 |
| Unadjusted annualized relapses rate ^{a} | 0.279 | 0.305 | 0.116 |
| | | | |

| Adjusted annualized relapse rate ^{b} | | | |
|---|---|---|---|
| Estimate (95% Cl) | 0.260 (0.108, 0.625) | 0.280 (0.101, 0.774) | 0.109 (0.031, 0.388) |
| Relative risk (95% Cl) | | 1.079 (0.342, 3.403) | 0.420 (0.085, 2.063) |
| P-value | | 0.8968 | 0.2852 |
| | | | |

| Patient annualized relapse rate ^{c} | | | |
|---|---|---|---|
| N | 41 | 37 | 38 |
| Mean (SD) | 0.259 (0.703) | 0.360 (0.992) | 0.112 (0.482) |
| Median | 0.000 | 0.000 | 0.000 |
| Min : Max | 0.00 : 2.19 | 0.00 : 4.62 | 0.00 : 2.16 |

| | | | |
|---|---|---|---|
| ^{a} The total number of confirmed relapses that occurred during the study divided by the total number of patient-years followed in the study. ^{b} Poisson model with the total number of confirmed relapses onset between randomization date and last dose date as the response variable, treatment, IFN-β dose strata and region as covariates, and log-transformed standardized study duration as an offset variable. ^{c} The number of confirmed relapses for each patient divided by the number of years followed in the study for that patient. | | | |

### Example 2

Design/Methods: 86 of the116 patients that were randomized to treatment (placebo: 41; 7 mg: 37; 14 mg: 38) for the first 6-months in Example 1 completed this 6-month period and accepted to continue medication for an additional 6-month period (placebo: 31, 7 mg: 28, 14 mg: 27). Safety was evaluated from TEAE, physical exam (every 6 weeks), laboratory data (every 6 weeks), ECG (at the close-out visit), pancreatic ultrasound (at the close-out visit) and brain MRI (at the close-out visit). Relapses were recorded and EDSS was performed every 6-weeks. Annualized relapse rates (ARR) were estimated by a Poisson model adjusted to IFN strata and to geographical regions. The following results report the entire 48-week double-blind treatment period.

Results: Baseline characteristics were similar among groups: mean age 40 years, 70% female, mean EDSS 2.5. 40% of patients were relapse-free in the previous year and 33% of patients were on low-dose IFN-beta.

Safety: Eight TEAEs led to treatment discontinuation (placebo: 2; 7 mg: 3; 14 mg: 3). Hepatic disorder TEAEs were mainly asymptomatic liver enzyme increases. Six patients had ALT increased above 3ULN (placebo: 2; 7 mg: 1; 14 mg: 3) without a concomitant increase in bilirubin. The most frequently reported treatment emergent adverse events were upper respiratory tract infections as a whole (placebo: 17.1%; 7 mg: 16.2%; 14 mg: 23.7%), mainly nasopharyngitis and sinusitis, all type of headaches (placebo:7.3%; 7 mg: 5.4%; 14 mg: 18.4%), all gastrointestinal disorders (placebo: 24.4%; 7 mg: 18.9%; 14 mg: 31.6%). White blood cell counts decreases were numerically comparable in both teriflunomide and placebo treatment groups (placebo: 3; 7 mg: 3; 14 mg: 4) and no patients discontinued treatment due to neutropenia or infection.

Efficacy: As shown in Tables 4, 5 and 6, the adjusted results from the 48 week of study showed that patients taking 7 mg or 14 mg teriflunomide in addition to stable-dose IFN-beta experienced a significant relative risk reduction in the number of T1-Gd lesions on brain magnetic resonance imaging, 84.6% (p=0.0005) and 82.8% (p<0.0001), for 7 mg and 14 mg when added to IFN-beta respectively, compared with placebo added to IFN-beta. There was a trend to a dose-dependent relative reduction of T1-Gd lesion volume in both teriflunomide arms compared to placebo of 72.1% at 7 mg (p=0.1104) and 70.6% at 14 mg (p=0.0154). There was also a dose-dependent trend to a reduction in annualized relapse rate of 32.6% (p=0.43) and 57.9% (p=0.11) in 7 mg or 14 mg teriflunomide groups respectively compared to IFN-beta. The proportion of patients free of T1-Gd lesions was higher in both teriflunomide arms (placebo: 55.3%; 7 mg: 69.4%; 14 mg: 76.3%).

Conclusion: In this one-year study, the addition of teriflunomide to stable-dosed IFN-beta significantly improved disease control as evaluated by MRI activity beyond IFN-beta alone, and trended to a similar reduction in clinical relapse - all with good safety and tolerability.

**Table 4 - Total number of Gd-enhancing T1-lesions per MRI scan - ITT population**

| | | **teriflunomide** | |
|---|---|---|---|
| | **Placebo+IFN-β (N=41)** | **7 mg teri+IFN-β (N=37)** | **14 mg teri+IFN-β (N=38)** |
| **Baseline** | | | |
| Patients with >=1 Gd-enhancing T1-lesions | | | |
| Yes | 9 (22.5%) | 8 (21.6%) | 8 (21.1%) |
| No | 31 (77.5%) | 29 (78.4%) | 30 (78.9%) |
| | | | |

| Patient Gd-enhancing T1-lesions | | | |
|---|---|---|---|
| N | 40 | 37 | 38 |
| Mean (SD) | 0.725 (1.679) | 0.838 (2.892) | 0.579 (1.734) |
| Median | 0.000 | 0.000 | 0.000 |
| Min : Max | 0.00 : 7.00 | 0.00 : 17.00 | 0.00: 10.00 |
| | | | |

| **Post-baseline** | | | |
|---|---|---|---|
| Patients with >=1 Gd-enhancing T1-lesions | | | |
| Yes | 17 (44.7%) | 11 (30.6%) | 9 (23.7%) |
| No | 21 (55.3%) | 25 (69.4%) | 29 (76.3%) |
| P-value ^{a} | | 0.2381 | 0.0896 |
| | | | |

| Number of Gd-enhancing T1-lesions | | | |
|---|---|---|---|
| 0 | 21 (55.3%) | 25 (69.4%) | 29 (76.3%) |
| 1 | 4 (10.5%) | 8 (22.2%) | 6 (15.8%) |
| 2 | 2 (5.3%) | 0 | 1 (2.6%) |
| 3 | 2 (5.3%) | 0 | 1 (2.6%) |
| >=4 | 9 (23.7%) | 3 (8.3%) | 1 (2.6%) |
| | | | |
| Total number of Gd-enhancing T1-lesions | 101 | 35 | 17 |
| Total number of scans | 144 | 123 | 132 |
| Unadjusted Gd-enhancing T1-lesions per scan ^{b} | 0.701 | 0.285 | 0.129 |
| | | | |

| Adjusted Gd-enhancing T1-lesions per scan ^{c} | | | |
|---|---|---|---|
| Estimate (95% Cl) | 0.521 (0.318, 0.854) | 0.080 (0.032, 0.204) | 0.090 (0.052, 0.154) |
| Relative risk (95% Cl) | | 0.154 (0.053, 0.445) | 0.172 (0.092, 0.323) |
| P-value | | 0.0005 | <.0001 |
| | | | |

| Patient Gd-enhancing T1-lesions per scan ^{d} | | | |
|---|---|---|---|
| N | 38 | 36 | 38 |
| Mean (SD) | 0.729 (1.305) | 0.372 (1.106) | 0.138 (0.369) |
| Median | 0.000 | 0.000 | 0.000 |
| Min : Max | 0.00 : 4.60 | 0.00 : 5.00 | 0.00 : 2.00 |

| | | | |
|---|---|---|---|
| ^{a} From Fisher's exact test. ^{b} The total number of Gd-enhancing T1-lesion that occurred during the study divided by the total number of scans during the study. ^{c} Poisson model with the total number of Gd-enhancing T1-lesions as the response variable, baseline number of Gd-enhancing T1-lesions, treatment, IFN-beta dose stratum, and region as covariates, and log-transformed number of scans as an offset variable. ^{d} The number of Gd-enhancing T1-lesions for each patient divided by the number of scans for that patient. | | | |

**Table 5 - Total volume (ml) of Gd-enhancing T1-lesions per MRI scan - ITT population**

| | | **teriflunomide** | |
|---|---|---|---|
| | **Placebo+IFN-β (N=41)** | **7 mg teri+IFN-β (N=37)** | **14 mg teri+IFN-β (N=38)** |
| **Baseline** | | | |
| N | 40 | 37 | 38 |
| Mean (SD) | 0.078 (0.201) | 0.107 (0.344) | 0.047 (0.120) |
| Median | 0.000 | 0.000 | 0.000 |
| Min : Max | 0.00 : 0.94 | 0.00 : 1.67 | 0.00 : 0.54 |
| | | | |

| **Post-baseline** | | | |
|---|---|---|---|
| Gd-enhancing T1-lesions per scan ^{a} | 0.068 | 0.019 | 0.02 |
| | | | |

| Patient Gd-enhancing T1-lesions per scan ^{b} | | | |
|---|---|---|---|
| N | 38 | 36 | 38 |
| Mean (SD) | 0.067 (0.164) | 0.036 (0.148) | 0.023 (0.087) |
| Median | 0.000 | 0.000 | 0.000 |
| Min : Max | 0.00 : 0.88 | 0.00 : 0.87 | 0.00 : 0.50 |
| | | | |
| P-value ^{c} | | 0.1104 | 0.0154 |

| | | | |
|---|---|---|---|
| ^{a} The total volume of Gd-enhancing T1-lesion that occurred during the study divided by the total number of scans during the study. ^{b} The volume of Gd-enhancing T1-lesions for each patient divided by the number of scans for that patient. ^{c} Rank ANCOVA adjusted for IFN-beta dose strata, region and ranked baseline volume of Gd-enhancing T1-lesions. | | | |

**Table 6 - Analysis of MS relapse - ITT population**

| | | **teriflunomide** | |
|---|---|---|---|
| | **Placebo+IFN-β (N=41)** | **7 mg teri+IFN-β (N=37)** | **14 mg teri+IFN-β (N=38)** |
| Number of patients with >=1 relapses | | | |
| Yes | 8 (19.5%) | 7 (18.9%) | 5 (13.2%) |
| No | 33 (80.5%) | 30 (81.1%) | 33 (86.8%) |
| | | | |

| Number of relapses | | | |
|---|---|---|---|
| 0 | 33 (80.5%) | 30 (81.1%) | 33 (86.8%) |
| 1 | 4 (9.8%) | 6 (16.2%) | 5 (13.2%) |
| 2 | 3 (7.3%) | 1 (2.7%) | 0 |
| 3 | 1 (2.4%) | 0 | 0 |
| >=4 | 0 | 0 | 0 |
| | | | |
| Total number of relapses | 13 | 8 | 5 |
| Total patient-years followed | 31.9 | 28.1 | 29.5 |
| Unadjusted annualized relapses rate ^{a} | 0.408 | 0.285 | 0.169 |
| | | | |

| Adjusted annualized relapse rate ^{b} | | | |
|---|---|---|---|
| Estimate (95% Cl) | 0.343 (0.162, 0.727) | 0.231 (0.101, 0.529) | 0.144 (0.065, 0.318) |
| Relative risk (95% Cl) | | 0.674 (0.250, 1.816) | 0.421 (0.150, 1.182) |
| P-value | | 0.4355 | 0.1005 |
| | | | |

| Patient annualized relapse rate ^{c} | | | |
|---|---|---|---|
| N | 41 | 37 | 38 |
| Mean (SD) | 0.356 (0.858) | 0.383 (0.992) | 0.167 (0.465) |
| Median | 0.000 | 0.000 | 0.000 |
| Min : Max | 0.00: 4.12 | 0.00: 4.62 | 0.00 : 2.16 |

| | | | |
|---|---|---|---|
| ^{a} The total number of confirmed relapses that occurred during the study divided by the total number of patient-years followed in the study. ^{b} Poisson model with the total number of confirmed relapses onset between randomization date and last dose date as the response variable, treatment, IFN-beta dose stratum, and region as covariates, and log-transformed standardized study duration as an offset variable. ^{c} The number of confirmed relapses for each patient divided by the number of years followed in the study for that patient. | | | |

## Claims

1. Teriflunomide for use in treating relapsing-remitting form of multiple sclerosis, in a patient in need thereof, said use comprising administering to the patient about 7 mg or about 14 mg teriflunomide, and a pharmaceutically effective amount of interferon beta-1a or 1b.

2. Teriflunomide for use according to claim 1, wherein said use comprises administering to the patient about 7 mg or about 14 mg teriflunomide once a day, and a stable dose of interferon beta-1 a or 1b.

3. Teriflunomide for use according to claim 2, said use comprising administering to the patient about 7 mg or about 14 mg teriflunomide once a day, and a stable dose of interferon beta-1 a or 1 b in a clinically proven effective method.

4. Teriflunomide for use in reducing the number of T1-Gd lesions in a patient afflicted with relapsing-remitting form of multiple sclerosis, said use comprising administering to the patient about 7 mg or about 14 mg teriflunomide, and a pharmaceutically effective amount of interferon beta-1a or 1 b.

5. Teriflunomide for use according to claim 4, wherein said use comprises administering to the patient about 7 mg or about 14 mg teriflunomide once a day, and a stable dose of interferon beta-1a or 1 b.

6. Teriflunomide for use according to claim 5, said use comprising administering to the patient about 7 mg or about 14 mg teriflunomide once a day, and a stable dose of interferon beta-1a or 1 b in a clinically proven effective method.

7. Teriflunomide for use in reducing the number of T1-Gd lesions in a patient afflicted with relapsing-remitting form of multiple sclerosis, said use comprising administering to the patient about 7 mg or about 14 mg teriflunomide, and a stable dose of interferon beta-1a or 1b, wherein more T1-Gd lesions are reduced in the patient than when the patient is administered a stable dose of interferon beta-1 a or 1 b alone.

8. Teriflunomide for use according to claim 6, said use comprising administering to the patient about 7 mg or about 14 mg teriflunomide once a day, and a stable dose of interferon beta-1a or 1b in a clinically proven effective method, wherein more T1-Gd lesions are reduced in the patient than when the patient is administered a stable dose of interferon beta-1a or 1 b alone.

9. Teriflunomide for use according to claim 5, said use comprising administering to the patient about 7 mg teriflunomide once a day, and a stable dose of interferon beta-1 a or 1b, wherein the number of T1-Gd lesions in the patients is reduced about 82.6% to about 84.6% comparing to the number of lesions in patients treated by a stable dose of interferon beta-1 a or 1 b alone.

10. Teriflunomide for use according to claim 5, said use comprising administering to the patient about 14 mg teriflunomide once a day, and a stable dose of interferon beta-1a or 1b, wherein the number of T1-Gd lesions in the patients is reduced about 82.8% to about 84.4% comparing to the number of lesions in patients treated by a stable dose of interferon beta-1 a or 1 b alone.

11. Teriflunomide for use in reducing the volume of T1-Gd lesions in a patient afflicted with relapsing-remitting form of multiple sclerosis, said use comprising administering to the patient about 7 mg or about 14 mg teriflunomide once a day, and a stable dose of interferon beta-1 a or 1 b.

12. Teriflunomide for use according to claim 11, said use comprising administering to the patients about 7 mg or about 14 mg teriflunomide once a day, and a stable dose of interferon beta-1a or 1b in a clinically proven effective method.

13. Teriflunomide for use according to claim 11, said use comprising administering to the patient about 7 mg or about 14 mg teriflunomide once a day, and a stable dose of interferon beta-1a or 1b, wherein more volume of T1-Gd lesions is reduced in the patient treated by the method than in a patient treated by a stable dose of interferon beta-1a or 1 b alone.

14. Teriflunomide for use according to claim 11, said use comprising administering to the patients about 14 mg teriflunomide once a day, and a stable dose of interferon beta-1a or 1b, wherein more volumes of T1-Gd lesions are reduced in the patients than in patients treated by a stable dose of interferon beta-1 a or 1 b alone in a clinically proven effective method.

15. Teriflunomide for use according to claim 11, said use comprising administering to the patient about 14 mg teriflunomide once a day, and a stable dose of interferon beta-1a or 1b, wherein the volume of T1-Gd lesions in the patients treated by the method is reduced about 64.7% to about 70.6% comparing to the volume of lesions in patients treated by a stable dose of interferon beta-1a or 1 b alone.

16. Teriflunomide for use according to claim 2, said use comprising administering to the patients 7 mg teriflunomide once a day and a stable dose of interferon beta-1 a or 1 b, wherein about 69.4% of the patients are free of T1-Gd lesions after about 24-week treatment.

17. Teriflunomide for use according to claim 2, said use comprising administering to the patients 7 mg teriflunomide once a day and a stable dose of interferon beta-1a or 1b, wherein about 69.4% of the patients are free of T1-Gd lesions after about 48-week treatment.

18. Teriflunomide for use according to claim 2, said use comprising administering to the patients 14 mg teriflunomide once a day and a stable dose of interferon beta-1a or 1b, wherein about 81.6% of the patients are free of T1-Gd lesions after about 24-week treatment.

19. Teriflunomide for use according to claim 2, said use comprising administering to the patients 14 mg teriflunomide once a day and a stable dose of interferon beta-1a or 1 b, wherein about 76.3% of the patients are free of T1-Gd lesions after about 48-week treatment.

## Patentansprüche

1. Teriflunomid zur Verwendung in der Behandlung der schubförmig verlaufenden Form der multiplen Sklerose bei einem Patienten, bei dem ein Bedürfnis daran besteht, wobei die Verwendung die Verabreichung von ungefähr 7 mg oder ungefähr 14 mg Teriflunomid und eine pharmazeutisch wirksame Menge von Interferon beta-1a oder-1b an den Patienten umfasst.

2. Teriflunomid zur Verwendung nach Anspruch 1, wobei die Verwendung die Verabreichung von ungefähr 7 mg oder ungefähr 14 mg Teriflunomid einmal pro Tag und einer stabilen Dosis von Interferon beta-1a oder-1b an den Patienten umfasst.

3. Teriflunomid zur Verwendung nach Anspruch 2, wobei die Verwendung die Verabreichung von ungefähr 7 mg oder ungefähr 14 mg Teriflunomid einmal pro Tag und einer stabilen Dosis von Interferon beta-1a oder-1b an den Patienten in einer klinisch belegten, wirksamen Methode umfasst.

4. Teriflunomid zur Verwendung in der Reduktion der Anzahl der T1-Gd-Läsionen bei einem Patienten, der an schubförmig verlaufender multipler Sklerose leidet, wobei die Verwendung die Verabreichung von ungefähr 7 mg oder ungefähr 14 mg Teriflunomid und eine pharmazeutisch wirksame Menge von Interferon beta-1a oder-1b an den Patienten umfasst.

5. Teriflunomid zur Verwendung nach Anspruch 4, wobei die Verwendung die Verabreichung von ungefähr 7 mg oder ungefähr 14 mg Teriflunomid einmal pro Tag und einer stabilen Dosis von Interferon beta-1a oder-1b an den Patienten umfasst.

6. Teriflunomid zur Verwendung nach Anspruch 5, wobei die Verwendung die Verabreichung von ungefähr 7 mg oder ungefähr 14 mg Teriflunomid einmal pro Tag und einer stabilen Dosis von Interferon beta-1a oder-1b an den Patienten in einer klinisch belegten, wirksamen Methode umfasst.

7. Teriflunomid zur Verwendung in der Reduktion der Anzahl der T1-Gd-Läsionen bei einem Patienten, der an schubförmig verlaufender multipler Sklerose leidet, wobei die Verwendung die Verabreichung von ungefähr 7 mg oder ungefähr 14 mg Teriflunomid und einer stabilen Dosis von Interferon beta-1a oder-1b an den Patienten umfasst, wobei bei dem Patienten mehr T1-Gd-Läsionen reduziert werden als wenn dem Patienten eine stabile Dosis von Interferon beta-1a oder-1b allein verabreicht wird.

8. Teriflunomid zur Verwendung nach Anspruch 6, wobei die Verwendung die Verabreichung von ungefähr 7 mg oder ungefähr 14 mg Teriflunomid einmal pro Tag und einer stabilen Dosis von Interferon beta-1a oder-1b an den Patienten in einer klinisch belegten, wirksamen Methode umfasst, wobei bei dem Patienten mehr T1-Gd-Läsionen reduziert werden als wenn dem Patienten eine stabile Dosis von Interferon beta-1a oder-1b allein verabreicht wird.

9. Teriflunomid zur Verwendung nach Anspruch 5, wobei die Verwendung die Verabreichung von ungefähr 7 mg Teriflunomid einmal pro Tag und einer stabilen Dosis von Interferon beta-1a oder-1b an den Patienten umfasst, wobei die Anzahl der T1-Gd-Läsionen bei dem Patienten um ungefähr 82,6% bis ungefähr 84,6% im Vergleich zu der Anzahl der Läsionen bei Patienten, die mit einer stabilen Dosis von Interferon beta-1a oder-1b allein behandelt werden, reduziert wird.

10. Teriflunomid zur Verwendung nach Anspruch 5, wobei die Verwendung die Verabreichung von ungefähr 14 mg Teriflunomid einmal pro Tag und einer stabilen Dosis von Interferon beta-1a oder-1b an den Patienten umfasst, wobei die Anzahl der T1-Gd-Läsionen bei dem Patienten um ungefähr 82,8% bis ungefähr 84,4% im Vergleich zu der Anzahl der Läsionen bei Patienten, die mit einer stabilen Dosis von Interferon beta-1a oder-1b allein behandelt werden, reduziert wird.

11. Teriflunomid zur Verwendung in der Reduktion des Volumens der T1-Gd-Läsionen bei einem Patienten, der an schubförmig verlaufender multipler Sklerose leidet, wobei die Verwendung die Verabreichung von ungefähr 7 mg oder ungefähr 14 mg Teriflunomid einmal pro Tag und einer stabilen Dosis von Interferon beta-1a oder-1b an den Patienten umfasst.

12. Teriflunomid zur Verwendung nach Anspruch 11, wobei die Verwendung die Verabreichung von ungefähr 7 mg oder ungefähr 14 mg Teriflunomid einmal pro Tag und einer stabilen Dosis von Interferon beta-1a oder-1b an den Patienten in einer klinisch belegten, wirksamen Methode umfasst.

13. Teriflunomid zur Verwendung nach Anspruch 11, wobei die Verwendung die Verabreichung von ungefähr 7 mg oder ungefähr 14 mg Teriflunomid einmal pro Tag und einer stabilen Dosis von Interferon beta-1a oder-1b an den Patienten umfasst, wobei bei dem mit der Methode behandelten Patienten mehr Volumen von T1-Gd-Läsionen reduziert wird als bei einem Patienten, der mit einer stabilen Dosis von Interferon beta-1a oder-1b allein behandelt wird.

14. Teriflunomid zur Verwendung nach Anspruch 11, wobei die Verwendung die Verabreichung von ungefähr 14 mg Teriflunomid einmal pro Tag und einer stabilen Dosis von Interferon beta-1a oder-1b an Patienten umfasst, wobei bei den Patienten mehr Volumina von T1-Gd-Läsionen reduziert werden als bei Patienten, die mit einer stabilen Dosis von Interferon beta-1a oder-1b allein in einer klinisch belegten, wirksamen Methode behandelt werden.

15. Teriflunomid zur Verwendung nach Anspruch 11, wobei die Verwendung die Verabreichung von ungefähr 14 mg Teriflunomid einmal pro Tag und einer stabilen Dosis von Interferon beta-1a oder-1b an den Patienten umfasst, wobei bei den mit der Methode behandelten Patienten das Volumen der T1-Gd-Läsionen im Vergleich zu den Volumen der Läsionen bei Patienten, die mit einer stabilen Dosis von Interferon beta-1a oder-1b allein behandelt wurden, um ungefähr 64,7% bis ungefähr 70,6% verringert ist.

16. Teriflunomid zur Verwendung nach Anspruch 2, wobei die Verwendung die Verabreichung von 7 mg Teriflunomid einmal pro Tag und einer stabilen Dosis von Interferon beta-1a oder-1b an die Patienten umfasst, wobei nach einer ungefähr 24-wöchigen Behandlung 69,4% der Patienten frei von T1-Gd-Läsionen sind.

17. Teriflunomid zur Verwendung nach Anspruch 2, wobei die Verwendung die Verabreichung von 7 mg Teriflunomid einmal pro Tag und einer stabilen Dosis von Interferon beta-1a oder-1b an die Patienten umfasst, wobei nach einer ungefähr 48-wöchigen Behandlung 69,4% der Patienten frei von T1-Gd-Läsionen sind.

18. Teriflunomid zur Verwendung nach Anspruch 2, wobei die Verwendung die Verabreichung von 14 mg Teriflunomid einmal pro Tag und einer stabilen Dosis von Interferon beta-1a oder-1b an die Patienten umfasst, wobei nach einer ungefähr 24-wöchigen Behandlung 81,6% der Patienten frei von T1-Gd-Läsionen sind.

19. Teriflunomid zur Verwendung nach Anspruch 2, wobei die Verwendung die Verabreichung von 14 mg Teriflunomid einmal pro Tag und einer stabilen Dosis von Interferon beta-1a oder-1b an die Patienten umfasst, wobei nach einer ungefähr 48-wöchigen Behandlung 76,3% der Patienten frei von T1-Gd-Läsionen sind.

## Revendications

1. Tériflunomide pour utilisation dans le traitement de la forme récurrente-rémittente de la sclérose en plaques, chez un patient nécessitant cela, ladite utilisation comprenant l'administration au patient d'environ 7 mg ou environ 14 mg de tériflunomide, et une quantité pharmaceutiquement efficace d'interféron bêta-1a ou 1b.

2. Tériflunomide pour utilisation selon la revendication 1, ladite utilisation comprenant l'administration au patient d'environ 7 mg ou environ 14 mg de tériflunomide une fois par jour, et une dose stable d'interféron bêta-1a ou 1b.

3. Tériflunomide pour utilisation selon la revendication 2, ladite utilisation comprenant l'administration au patient d'environ 7 mg ou environ 14 mg de tériflunomide une fois par jour, et une dose stable d'interféron bêta-1a ou 1b dans un procédé efficace cliniquement éprouvé.

4. Tériflunomide pour utilisation dans la réduction du nombre de lésions T1-Gd chez un patient atteint de la forme récurrente-rémittente de la sclérose en plaques, ladite utilisation comprenant l'administration au patient d'environ 7 mg ou environ 14 mg de tériflunomide, et une quantité pharmaceutiquement efficace d'interféron bêta-1a ou 1b.

5. Tériflunomide pour utilisation selon la revendication 4, ladite utilisation comprenant l'administration au patient d'environ 7 mg ou environ 14 mg de tériflunomide une fois par jour, et une dose stable d'interféron bêta-1a ou 1b.

6. Tériflunomide pour utilisation selon la revendication 5, ladite utilisation comprenant l'administration au patient d'environ 7 mg ou environ 14 mg de tériflunomide une fois par jour, et une dose stable d'interféron bêta-1a ou 1b dans un procédé efficace cliniquement éprouvé.

7. Tériflunomide pour utilisation dans la réduction du nombre de lésions T1-Gd chez un patient atteint de la forme récurrente-rémittente de la sclérose en plaques, ladite utilisation comprenant l'administration au patient d'environ 7 mg ou environ 14 mg de tériflunomide, et une dose stable d'interféron bêta-1a ou 1b, plus de lésions T1-Gd étant réduites chez le patient que lorsque le patient reçoit une dose stable d'interféron bêta-1a ou 1b seule.

8. Tériflunomide pour utilisation selon la revendication 6, ladite utilisation comprenant l'administration au patient d'environ 7 mg ou environ 14 mg de tériflunomide une fois par jour, et une dose stable d'interféron bêta-1a ou 1b dans un procédé efficace cliniquement éprouvé, plus de lésions T1-Gd étant réduites chez le patient que lorsque le patient reçoit une dose stable d'interféron bêta-1a ou 1b seule.

9. Tériflunomide pour utilisation selon la revendication 5, ladite utilisation comprenant l'administration au patient d'environ 7 mg de tériflunomide une fois par jour, et une dose stable d'interféron bêta-1a ou 1b, le nombre de lésions T1-Gd chez les patients étant réduit d'environ 82,6 % à environ 84,6 % par rapport au nombre de lésions chez des patients traités par une dose stable d'interféron bêta-1a ou 1b seule.

10. Tériflunomide pour utilisation selon la revendication 5, ladite utilisation comprenant l'administration au patient d'environ 14 mg de tériflunomide une fois par jour, et une dose stable d'interféron bêta-1a ou 1b, le nombre de lésions T1-Gd in les patients étant réduit d'environ 82,8 % à environ 84,4 % par rapport au nombre de lésions chez des patients traités par une dose stable d'interféron bêta-1a ou 1b seule.

11. Tériflunomide pour utilisation dans la réduction du volume de lésions T1-Gd chez un patient atteint de la forme récurrente-rémittente de la sclérose en plaques, ladite utilisation comprenant l'administration au patient d'environ 7 mg ou environ 14 mg de tériflunomide une fois par jour, et une dose stable d'interféron bêta-1a ou 1b.

12. Tériflunomide pour utilisation selon 1a revendication 11, ladite utilisation comprenant l'administration aux patients d'environ 7 mg ou environ 14 mg de tériflunomide une fois par jour, et une dose stable d'interféron bêta-1a ou 1b dans un procédé efficace cliniquement éprouvé.

13. Tériflunomide pour utilisation selon la revendication 11, ladite utilisation comprenant l'administration au patient d'environ 7 mg ou environ 14 mg de tériflunomide une fois par jour, et une dose stable d'interféron bêta-1a ou 1b, plus de volume de lésions T1-Gd étant réduit chez le patient traité par le procédé que chez un patient traité par une dose stable d'interféron bêta-1a ou 1b seule.

14. Tériflunomide pour utilisation selon la revendication 11, ladite utilisation comprenant l'administration aux patients d'environ 14 mg de tériflunomide une fois par jour, et une dose stable d'interféron bêta-1a ou 1b, plus de volumes de lésions T1-Gd étant réduits chez les patients que chez les patients traités par une dose stable d'interféron bêta-1a ou 1b seule dans un procédé efficace cliniquement éprouvé.

15. Tériflunomide pour utilisation selon la revendication 11, ladite utilisation comprenant l'administration au patient d'environ 14 mg de tériflunomide une fois par jour, et une dose stable d'interféron bêta-1a ou 1b, le volume de lésions T1-Gd chez les patients traités par le procédé étant réduit d'environ 64,7 % à environ 70,6 % par rapport au volume de lésions chez des patients traités par une dose stable d'interféron bêta-1a ou 1b seule.

16. Tériflunomide pour utilisation selon la revendication 2, ladite utilisation comprenant l'administration aux patients de 7 mg de tériflunomide une fois par jour et une dose stable d'interféron bêta-1a ou 1b, environ 69,4 % des patients étant exempts de lésions T1-Gd après environ 24 semaines de traitement.

17. Tériflunomide pour utilisation selon la revendication 2, ladite utilisation comprenant l'administration aux patients de 7 mg de tériflunomide une fois par jour et une dose stable d'interféron bêta-1a ou 1b, environ 69,4 % des patients étant exempts de lésions T1-Gd après environ 48 semaines de traitement.

18. Tériflunomide pour utilisation selon la revendication 2, ladite utilisation comprenant l'administration aux patients de 14 mg de tériflunomide une fois par jour et une dose stable d'interféron bêta-1a ou 1b, environ 81,6 % des patients étant exempts de lésions T1-Gd après environ 24 semaines de traitement.

19. Tériflunomide pour utilisation selon la revendication 2, ladite utilisation comprenant l'administration aux patients de 14 mg de tériflunomide une fois par jour et une dose stable d'interféron bêta-1a ou 1b, environ 76,3 % des patients étant exempts de lésions T1-Gd après environ 48 semaines de traitement.
